(19) Europäisches Patentamt
European Patent Office
Office européen des brevets

(11) Publication number: **0 057 320**
**B1**

(12) **EUROPEAN PATENT SPECIFICATION**

(45) Date of publication of patent specification: **05.06.85**

(51) Int. Cl.$^4$: **B 01 J 27/18,** B 01 J 23/89, C 07 C 51/377

(21) Application number: **81305782.5**

(22) Date of filing: **08.12.81**

(54) **Mixed metal phosphorus oxide catalysts for the oxidative dehydrogenation of carboxylic acids utilizing them and preparation thereof.**

(30) Priority: **31.12.80 US 221859**

(43) Date of publication of application:
**11.08.82 Bulletin 82/32**

(45) Publication of the grant of the patent:
**05.06.85 Bulletin 85/23**

(84) Designated Contracting States:
**DE FR GB IT NL**

(56) References cited:
**EP-A-0 000 617**
**DE-A-2 624 555**
**FR-A-1 440 661**
**US-A-4 086 290**

(73) Proprietor: **THE STANDARD OIL COMPANY**
**Midland Building**
**Cleveland, Ohio 44115 (US)**

(72) Inventor: **Pedersen, Svend Erik**
**6277 Firwood Drive**
**Mentor Ohio 44060 (US)**
Inventor: **Callahan, James Louis**
**R.D. 6 Township Road, No. 101**
**Wooster Ohio 44691 (US)**
Inventor: **Hardman, Harley Foch**
**4989 Delevan Drive**
**Lyndhurst Ohio 44124 (US)**

(74) Representative: **Smith, Sydney et al**
**Elkington and Fife High Holborn House 52/54**
**High Holborn**
**London WC1V 6SH (GB)**

**EP 0 057 320 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the catalytic, oxidative dehydrogenation of saturated carboxylic acids to their corresponding unsaturated acids and to catalysts useful for this purpose. More particularly, it is directed to the production of unsaturated carboxylic acids such as methacrylic acid from saturated carboxylic acids such as isobutyric acid utilizing promoted iron phosphorus oxide catalysts.

The production of unsaturated carboxylic acids from their corresponding saturated acids using iron phosphorus oxide catalysts, with or without various promoters, is disclosed in the art.

U.S. Patent No. 3,948,959 discloses the preparation of unsaturated acids by oxidation of the corresponding saturated acid using iron phosphorus oxide catalysts promoted with Li, Na, K, Rb, Cs, Mg, Ca, Sr and Ba. U.S. Patent Nos. 3,634,494; 3,652,654; 3,855,279; 3,917,673 and 4,029,695 disclose the preparation of unsaturated acids and esters from saturated acids and esters using iron phosphorus oxide catalysts, containing bismuth and/or lead promoters, optionally with other promoter elements, including Mn, U, Pr, Ca, Sr and Cr. Prior art catalysts characteristically have exhibited short life and thermal instability.

In FR—A 1,440,661 there is described a process for the preparation of unsaturated aldehydes by the catalytic oxidation in the vapour phase of a hydrocarbon of the formula:

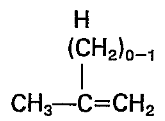

The catalyst used in this process comprises a mixture of phosphates of Ag, Cu or Fe in the molar ratio of Ag 1, Cu 0—2 and Fe 0—2 at least one of the elements Cu and Fe being present in a molar ratio of not less than 0.5 based on Ag.

We have found that unsaturated acids, particularly methacrylic acid, can be produced from their corresponding saturated acids, such as isobutyric acid, using iron phosphorus oxide catalysts promoted with particular elements, in increased yields corresponding to an increase of catalyst activity and/or selectivity over prior art catalysts. The catalysts of the present invention exhibit increased life and thermal stability with respect to the prior art catalysts.

Although promoters such as Mn, U, Cr, were disclosed as being suitable promoters in an iron lead mixed phosphate system if present in low amounts, and promoters such as Co, Ni, Cu, Zn, Cd and Ce were disclosed for that system as less than suitable promoters, we have found that these elements exhibit excellent promotional activity in the iron phosphorus oxide system for the oxydehydrogenation of saturated carboxylic acids wherein the iron phosphorus oxide is free of lead phosphate or oxide.

The present invention provides a process for the preparation of unsaturated acids by contacting their corresponding saturated acids with molecular oxygen or an oxygen-containing gas in the vapour phase, at a reaction temperature of 250°C to 600°C in the presence of a catalyst having the empirical formula

$$A_aFe_bP_cD_dO_x$$

wherein

A is selected from Al, B, Be, Cd, Co, Cr, Ga, Ge, In, Ni, Te, Th, Ti, Tl, U, V, Zn, Zr, rare earths and mixtures thereof, wherein D is selected from Ag, Cu, Mn and mixtures thereof,

and wherein

a=0—1.0
b=0.75—1.5
c=1.0—2.0
d=0—2.0
a+d is greater than zero

and

x is the number of oxygens needed to satisfy the valence requirements of the remaining elements.

Preferably a equals 0.15—0.5 and d equals 0.2—1.5.

There are also provided as new oxydehydrogenation catalyst compositions, compositions of the formula

$$A_aFe_bP_cD_dO_x$$

wherein

A is selected from Cd, Cr, Ge, Te, Th, Ti, U, V, Zr, rare earths and mixtures thereof;

wherein

D is selected from Ag, Cu, Mn and mixtures thereof;

and wherein

a=0—1.0;

b=0.75—1.5;

c=1.0—2.0; and

d=0—2.0; provided that a+d is greater than zero and when D is Ag or a mixture of Ag and Cu a is greater than zero;

and

x is the number of oxygens needed to satisfy the valence requirements of the remaining elements.

Preferably a equals 0.15—0.5 and d equals 0.2—1.5. Preferred rare earth metal promoters are La, Ce, Nd, Sm, Eu, Dy, Ho, Tm, Yb and Lu. Amongst the other elements for A, thorium is preferred.

In the process of the invention, saturated carboxylic acids are oxidatively dehydrogenated in the vapour phase, in the presence of promoted iron phosphorus oxide catalysts to form the corresponding unsaturated acid. The saturated acids preferably correspond to the formula

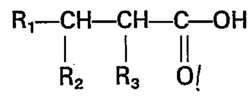

wherein $R_1$, $R_2$, and $R_3$ are each independently selected from the group consisting of hydrogen and alkyl groups containing 1 to 4 carbon atoms. The acids may contain other functional groups such as aryl or nitrile, provided the functional groups do not interfere with the dehydrogenation reaction, under the reaction conditions required. The dehydrogenation occurs essentially in the alpha, beta position.

The process of the present invention is highly suitable for the oxidative dehydrogenation of isobutyric acid to methacrylic acid.

The promoted iron phosphorus oxide catalysts of the present invention may be prepared according to methods known in the art.

One method of preparing the catalysts of the present invention includes introducing a compound of iron, and a compound containing the promoter element into water and contacting with a phosphorus compound, or the iron and promoter containing compound are introduced into an aqueous solution of phosphoric acid. Preferably, the compounds used containing iron and the promoter elements are soluble in water, and may include salts such as nitrates, halides, sulfates, acetates, carbonates, formates and the like. The resulting solution or slurry is evaporated to dryness, and the resulting solid is calcined at from 300° to 700°C. Alternatively, the catalyst may be prepared in an organic liquid medium. Alternatively, the aqueous solution or slurry can be adjusted to a pH of 5—6 before drying.

The catalyst may be formed into tablets, pellets or like shapes, and may be prepared for use in either fixed or fluid beds. The catalyst may be combined with inert diluents such as silica. Alternately, the catalyst may be coated upon inert supports, such as silica, alumina, alumina-silica, silicon carbide, titania, zirconia, zeolites and clays such as kieselguhr. Techniques of costing are described in U.S. Patent No. 4,077,912. The inert supports preferably are of at least 20 micrometers in diameter.

The promoted iron phosphorus oxide catalysts of the present invention exhibit enhanced activity and selectivity for the oxydehydrogenation of saturated carboxylic acids, particularly isobutyric acid. The catalysts also exhibit long life and thermal stability.

The saturated acids are contacted with the catalyst in the vapour phase, together with molecular oxygen. The molecular oxygen is most conveniently added as air, but synthetic streams containing oxygen are also suitable. In addition to the carboxylic acid feed and molecular oxygen, other gases may be added to the reactant feed. For example, steam is preferably added to the reactant feed to aid in the reaction, although the mechanism by which it does so is not certain. Inert diluents such as nitrogen, carbon monoxide, carbon dioxide and argon may also be added.

The molar ratios of the reactants may vary widely and are not critical. The ratios of carboxylic acid: air: steam are in the range of 1:2.5—50:0—50 and are preferably 1:3—10:10—30. Diluents may be present in the range of 0—40 moles per mole of carboxylic acid.

The reaction temperature may vary widely and is dependent upon the particular carboxylic acid and catalyst employed. Normally, temperatures of 250° to 600°C are employed with temperatures of 325°—450°C being preferred.

The contact time may vary from a fraction of a second to 50 seconds. In fixed bed reactions the contact time is preferably 0.5 seconds to 10 seconds, for fluid bed, preferably from 2 seconds to 20 seconds. The reaction may be conducted at atmospheric, superatmospheric or subatmospheric pressure, preferably from 1 psia (0.07 kg/cm$^2$) to 100 psia (7 kg/cm$^2$), most preferably between 10 (0.7 kg/cm$^2$) to 30 psia (2.10 kg/cm$^2$).

In the production of methacrylic acid from isobutyric acid, the major by-product is acetone (generally about 5—15% yield) which may be removed from the product by conventional methods.

The following Examples illustrate the invention.

Catalysts tested in the below examples were prepared according to the following procedure.

$Fe(NO_3)_3 . 9H_2O$, the appropriate promoter metal nitrate and $H_3PO_4$ (85%) were added to water in the amounts necessary to provide the molar ratios set forth in the tables below, for each of the particular catalysts reported. The solution of the components was evaporated to a dry paste with heating and stirring. The paste was dried for 16 hours at 110°C, and the resulting solid was calcined for 2 hours at 540°C. The solid was crushed and screened to 14—30 mesh (0.595—1.41 mm).

The catalyst particles were processed according to the examples below, and were tested for the oxydehydrogenation of isobutyric acid to methacrylic acid in a 20 cc fixed bed reactor. The reactor consisted of a length of stainless steel tubing having an outer diameter of 1.3 cm, and containing a full length 0.31 cm diameter axial thermowell. The reactor was heated with a split stainless steel block furnace.

The isobutyric acid was fed to the reactor by passing air through a saturator filled with isobutyric acid and maintained at a temperature of 108°C. Water was fed by means of a tubing pump and vapourized in a compartment maintained at 154°C before entering the reactor. Liquid products were analyzed on a Hewlett Packard 5710 A F.I.D. gas chromotograph. Gaseous products were analyzed on a conventional split column system.

The test reactions were run at atmospheric pressure, unless otherwise noted. Reaction conditions such as temperature, feed ratios, contact time and catalyst working rate (WWH=weight of isobutyric acid/weight of catalyst/hour) are listed in the tables below. Results of the tests reported in the tables below are reported in terms as follows:

$$\text{Single Pass Yield} = \frac{\text{Moles of Methacrylic Acid Formed} \times 100}{\text{Moles of Isobutyric Acid Fed}}$$

$$\text{Total Conversion} = \frac{\text{Moles of Isobutyric Acid Reacted} \times 100}{\text{Moles of Isobutyric Acid Fed}}$$

$$\text{Selectivity} = \frac{\text{Single Pass Yield} \times 100}{\text{Total Conversion}}$$

100% Active catalysts

Examples 1—13

Iron phosphorus oxide catalysts promoted with manganese (added as manganese nitrate) in varying molar ratios were prepared according to the procedure set forth above. The crushed and screened catalysts were charged to the reactor. The results of the tests, together with the amount of manganese in the catalysts are reported in Table I.

Examples 14—39

Iron phosphorus oxide catalysts promoted with silver (added as silver nitrate) in varying amounts were prepared according to the procedure set forth above. The crushed and screened catalysts were charged to the reactor. The results of the tests, together with the amount of silver in the catalysts are reported in Table II.

Examples 40—50

Iron phosphorus oxide catalysts promoted with copper (added as copper nitrate) in varying amounts were prepared according to the procedure set forth above. The crushed and screened catalysts were charged to the reactor. The results of the tests, together with the amount of copper in the catalysts are reported in Table III.

Examples 51—62

Catalysts of the formula $Th_{0.2}Fe_1P_{1.84}O_x$ were prepared from iron and thorium nitrates according to the procedure set forth above. The crushed and screened catalysts were charged to the reactor. The results of the tests are reported in Table IV.

Examples 63—118

Catalysts having the formula $A_aFe_1P_{1.84}O_x$ were prepared with the designated promoter metal nitrates as set forth in the procedure above. The crushed and screened catalysts were charged to the reactor. The results of the tests and the amount and identity of the promoter metal are reported in Table V.

Examples 119—124

Catalysts having the formula $A_aFe_1P_{1.84}D_dO_x$ containing two promoter elements were prepared as set forth in the procedure above. The crushed and screened catalysts were charged to the reactor. The results of the tests and the amounts and identities of the promoters are reported in Table VI.

Diluent-containing catalysts

Examples 125—132

Catalysts having the formula $Ag_{0.8}Fe_1P_{1.84}O_x$ were prepared according to the procedure set forth above., with the addition of various amounts of silica to the catalyst during preparation by slurrying in water. The crushed and screened catalysts were charged to the reactor. The results of the tests and the weight percents of active material and silica are reported in Table VII.

Coated catalysts

Examples 133—140

Catalysts of the formula $A_aFe_{1.0}P_{1.84}O_x$ were prepared according to the procedure set forth above. The crushed and screened catalysts were ground to a fine powder, and coated upon Alundum SA 5209 spheres (trade designation of Norton Company) according to the method set forth in U.S. Patent No. 4,077,912, with water as the wetting agent. The results of the tests, the catalyst used and the weight percent of catalyst loading (based upon total weight) are reported in Table VIII.

Examples 141—150

Catalysts of the formula $A_{0.6}Fe_{1.0}P_{1.8}O_x$ were prepared according to the procedure set forth above, except that the pH of the aqueous solution was adjusted to about 6 by addition of concentrated $NH_4OH$ solution. The dried catalysts were ground to a fine powder and coated on Alundum which had been ground to 10—20 mesh, (0.850—2.00 mm) using the method set forth in U.S. Patent No. 4,077,912 with absolute ethanol as a wetting agent. The coated particles were dried at 175°C for 30 minutes and calcined for 2 hours at 540°C. The catalyst loading was 17 weight percent.

The catalysts were tested in a 5 cc reactor consisting of a 6 1/2" (16.5 cm) stainless steel tube of 5/16" (0.8 cm) inner diameter immersed in a molten salt bath. Feed ratios were 1 IBA/5 AIR/25 $H_2O$ at a contact time of about 2 seconds. Reaction temperature was 415°C. Run time was one hour. Results of the tests and the identity of the promoter elements contained in the catalysts are listed in Table IX.

We have found that the iron phosphorus oxide catalysts, promoted according to the present invention, are not particularly suited to the oxydehydrogenation of saturated carboxylic acid esters, due to the hydrolysis of the ester by water formed as a by-product of the reaction even if steam is not co-fed. The oxydehydrogenation of the ester with these catalysts generally results either primarily in the formation of the unsaturated acid, or in low conversions to both acid and ester.

A catalyst of the formula $Th_{0.2}Fe_{1.0}P_{1.84}O_x$ was prepared according to the procedure of Examples 51—62. Methyl isobutyrate was oxydehydrogenated over this catalyst in the 20 cc reactor described above at 444°C with a feed ratio of MIBA/AIR/$H_2O$/$N_2$ equals 1/4.5/18/7.8 and a contact time of 1.6 seconds. Total conversion was 95.6%, but yield of methyl methacrylate was only 11.6% with a yield of methacrylic acid of 38.6%. With no steam being co-fed, (replaced by nitrogen), yield of methyl methacrylate increased to 23%, but total conversion decreased to 47.8%.

As is demonstrated by the test results reported in Tables I to IX, promoted iron phosphorus oxide catalysts according to the present invention exhibit high activity and selectivity in the oxydehydrogenation of saturated carboxylic acids, particularly isobutyric acid, to the corresponding unsaturated acid. The catalysts of the invention additionally exhibit long life and thermal stability, as is demonstrated by the test results reported in the tables.

TABLE I

Oxydehydrogenation of isobutyric acid to methacrylic acid over $Mn_aFe_{1.0}P_{1.84}O_x$ catalysts

| Example No. | Molar ratio-Mn(a) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 1 | 0.33 | 1/4.8/24/7 | 429 | 0.9 | 0.3 | 58.7 | 64.1 | 91.6 | 22 |
| 2 | 0.33 | 1/4.8/24.7 | 447 | 0.9 | 0.3 | 54.9 | 57.0 | 96.4 | 41 |
| 3 | 0.33 | 1/4.1.24.7 | 410 | 1.0 | 0.34 | 76.1 | 76.9 | 98.9 | 22.5 |
| 4 | 0.33 | 1/4.1.24.7 | 410 | 1.0 | 0.34 | 74.6 | 75.4 | 98.8 | 47 |
| 5 | 0.5 | 1/4.4/24.7 | 393 | 1.0 | 0.34 | 76.2 | 77.6 | 98.3 | 36 |
| 6 | 0.5 | 1/3.4/24.7 | 390 | 1.0 | 0.34 | 76.7 | 81.8 | 93.8 | 58.5 |
| 7 | 0.5 | 1/3.7/24.7 | 419 | 1.0 | 0.34 | 79.3 | 81.5 | 97.3 | 114.5 |
| 8 | 0.7 | 1/4.1/24.7 | 368 | 1.0 | 0.51 | 77.8 | 79.7 | 97.5 | 24.5 |
| 9 | 0.7 | 1/3.3/24.7 | 397 | 1.0 | 0.51 | 77.8 | 81.5 | 95.4 | 50.5 |
| 10 | 0.7 | 1/3.7/24.7 | 409 | 1.0 | 0.51 | 74.9 | 79.1 | 94.7 | 73.5 |
| 11 | 1.0 | 1/4.7/32.3 | 399 | 0.7 | 0.40 | 67.9 | 70.8 | 95.9 | 22 |
| 12 | 1.0 | 1/4.7/37.3 | 389 | 0.7 | 0.40 | 68.2 | 69.7 | 93.5 | 45 |
| 13 | 1.0 | 1/4.7/25.3 | 413 | 0.9 | 0.40 | 63.8 | 69.1 | 92.4 | 54 |

TABLE II

Oxydehydrogenation of isobutyric acid to methacrylic acid over $Ag_aFe_{1.0}P_{1.84}O_x$ catalysts

| Example No. | Molar ratio-Ag(a) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 14 | 0.33 | 1/4.7/24/7 | 385 | 1.0 | 0.23 | 67.7 | 75.3 | 89.9 | 24.4 |
| 15 | 0.33 | 1/4.8/24.7 | 382 | 1.0 | 0.23 | 69.0 | 70.2 | 98.3 | 52.5 |
| 16 | 0.33 | 1/4.8/24.7 | 378 | 1.0 | 0.23 | 66.8 | 69.2 | 96.6 | 73.6 |
| 17 | 0.66 | 1/4.9/25.3 | 406 | 0.9 | 0.27 | 71.9 | 71.9 | 100 | 24.6 |
| 18 | 0.66 | 1/4.4/25.3 | 382 | 1.0 | 0.27 | 74.3 | 76.1 | 97.5 | 50.9 |
| 19 | 0.70 | 1/4.5/25.3 | 387 | 1.0 | 0.18 | 73.8 | 76.1 | 96.9 | 45.9 |
| 20 | 0.75 | 1/4.8/24.7 | 368 | 1.0 | 0.20 | 74.9 | 80.8 | 92.7 | 24 |
| 21 | 0.75 | 1/5/24.7 | 388 | 1.0 | 0.20 | 80.0 | 81.1 | 98.7 | 36 |
| 22 | 0.75 | 1/5.5/25.6 | 391 | 0.9 | 0.20 | 80.2 | 82.1 | 97.6 | 108 |
| 23 | 0.75 | 1/5/17.3 | 407 | 1.2 | 0.20 | 70.6 | 72.9 | 96.8 | 198 |
| 24 | 0.75 | 1/5/30.5 | 390 | 0.8 | 0.20 | 75.1 | 76.8 | 97.8 | 243 |
| 25 | 0.8 | 1/4.5/24.7 | 388 | 1.0 | 0.19 | 78.4 | 79.6 | 98.5 | 46.5 |
| 26 | 0.8 | 1/3.6/24.7 | 395 | 1.0 | 0.19 | 80.4 | 82.2 | 97.8 | 83 |
| 27 | 0.8 | 1/4.7/24.7 | 406 | 1.0 | 0.19 | 73.1 | 73.1 | 100 | 156 |
| 28 | 0.8 | 1/4.8/24.7 | 383 | 1.0 | 0.19 | 73.1 | 75.1 | 97.3 | 244 |

TABLE II (cont'd)
$Ag_aFe_{1.0}P_{1.84}O_x$

| Example No. | Molar ratio-Ag(a) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 29 | 0.8 | 1/4.8/17.5 | 390 | 1.3 | 0.19 | 70.9 | 74.4 | 95.3 | 250 |
| 30 | 0.85 | 1/5.5/24.7 | 368 | 1.0 | 0.18 | 73.6 | 76.7 | 95.9 | 54.5 |
| 31 | 0.85 | 1/4.8/24.7 | 368 | 1.0 | 0.18 | 81.4 | 84.5 | 96.3 | 84 |
| 32 | 0.85 | 1/4.5/24.7 | 370 | 1.1 | 0.18 | 77.6 | 80.7 | 96.2 | 127 |
| 33* | 0.8 | 1/5/26.2 | 376 | 0.9 | 0.26 | 74.7 | 75.3 | 99.2 | 24 |
| 34* | 0.8 | 1/4.1/26.2 | 373 | 0.9 | 0.26 | 76.3 | 78.4 | 97.3 | 48.5 |
| 35* | 0.8 | 1/3.6/32.9 | 376 | 0.8 | 0.26 | 79.9 | 81.9 | 97.6 | 59.5 |
| 36 | 1.0 | 1/4.7/24.7 | 386 | 1.0 | 0.22 | 73.7 | 76.1 | 96.9 | 26.5 |
| 37 | 1.0 | 1/4.7/24.7 | 389 | 1.0 | 0.22 | 71.0 | 78.2 | 90.8 | 52 |
| 38 | 1.0 | 1/4.8/24.7 | 360 | 1.1 | 0.14 | 70.4 | 72.7 | 97.0 | 24 |
| 39 | 1.5 | 1/5/24.7 | 366 | 1.0 | 0.13 | 69.4 | 71.2 | 97.4 | 25.8 |

* Molar ratio Fe=1.2.

TABLE III

Oxydehydrogenation of isobutyric acid to methacrylic acid over $Cu_aFe_{1.0}P_{1.84}O_x$ catalysts

| Example No. | Molar ratio-Cu(a) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 40 | 0.2 | 1/4.8/24.7 | 405 | 1.0 | 0.27 | 68.1 | 72.1 | 94.4 | 24 |
| 41 | 0.2 | 1/4.8/24.7 | 387 | 1.0 | 0.27 | 70.0 | 72.1 | 97.1 | 46 |
| 42 | 0.33 | 1/4.8/24.7 | 396 | 1.0 | 0.28 | 66.2 | 66.3 | 99.9 | 27 |
| 43 | 0.33 | 1/4.9/24.7 | 386 | 1.0 | 0.28 | 67.1 | 71.2 | 94.3 | 72 |
| 44* | 0.33 | 1/4.3/22.0 | 374 | 1.7 | 0.32 | 65.7 | 69.9 | 94.0 | 107 |
| 45 | 0.66 | 1/4.8/24.7 | 368 | 1.0 | 0.26 | 70.9 | 72.9 | 97.3 | 25 |
| 46 | 0.66 | 1/4.8/24.7 | 368 | 1.0 | 0.26 | 71.4 | 74.0 | 96.6 | 49 |
| 47* | 0.66 | 1/4.8/13.0 | 410 | 0.9 | 0.77 | 61.9 | 67.0 | 92.4 | 60 |
| 48* | 0.66 | 1/4.2/22.6 | 386 | 0.9 | 0.52 | 68.1 | 72.6 | 93.8 | 67 |
| 49* | 0.66 | 1/4.7/12.9 | 430 | 0.9 | 0.77 | 63.1 | 68.1 | 92.8 | 76 |
| 50 | 1.0 | 1/4.9/24.7 | 429 | 0.9 | 0.27 | 49.5 | 55.9 | 88.5 | 24 |

* Run at reactor outlet pressure 12 PSIG (1,84 $kg/cm^2$)

TABLE IV

Oxydehydrogenation of isobutyric acid to methacrylic acid over $Th_{0.2}Fe_{1.0}P_{1.84}O_x$ catalysts

| Example No. | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|
| 51 | 1/5/11 | 400 | 1.7 | 0.34 | 71.0 | 71.2 | 99.8 | 39 |
| 52 | 1/5/11 | 398 | 1.7 | 0.34 | 67.5 | 70.6 | 95.6 | 68 |
| 53 | 1/5/11 | 402 | 1.7 | 0.34 | 60.5 | 65.4 | 92.5 | 136 |
| 54 | 1/5/15 | 402 | 1.4 | 0.34 | 66.9 | 69.0 | 96.9 | 203 |
| 55 | 1/4/20 | 413 | 1.1 | 0.34 | 66.7 | 72.9 | 91.5 | 330 |
| 56 | 1/4/26 | 423 | 0.9 | 0.34 | 65.7 | 71.2 | 92.2 | 401 |
| 57 | 1/5.5/26 | 412 | 0.9 | 0.34 | 63.3 | 67.1 | 94.3 | 499 |
| 58 | 1/5/24 | 397 | 1.0 | 0.34 | 61.0 | 70.9 | 86.0 | 616 |
| 59 | 1/5/24 | 406 | 1.0 | 0.34 | 65.3 | 68.9 | 94.7 | 809 |
| 60 | 1/5/25 | 426 | 1.4 | 0.22 | 57.9 | 66.4 | 87.2 | 979 |
| 61 | 1/5/25 | 407 | 0.9 | 0.34 | 70.2 | 71.6 | 98.1 | 1,045 |
| 62 | 1/5/25 | 402 | 1.0 | 0.34 | 68.8 | 69.4 | 99.1 | 1,074 |

TABLE V

Oxydehydrogenation of isobutyric acid to methacrylic acid using $A_aFe_{1.0}P_{1.84}O_x$ catalysts

| Example No. | Promoter & mole ratio ($A_a$) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 63 | $Ce_{0.2}$ | 1/5/31 | 391 | 1.8 | 0.18 | 53.8 | 57.0 | 94.3 | 8 |
| 64 | $Ce_{0.2}$ | 1/8/50 | 390 | 1.8 | 0.11 | 58.7 | 59.7 | 98.3 | 16 |
| 65 | $Co_{0.33}$ | 1/4.8/24.7 | 428 | 0.9 | 0.28 | 61.0 | 65.1 | 93.8 | 19 |
| 66 | $Co_{0.33}$ | 1/4.8/24.7 | 446 | 0.9 | 0.28 | 61.9 | 64.0 | 96.6 | 55 |
| 67 | $Co_{0.33}$ | 1/3.9/26.1 | 432 | 0.9 | 0.29 | 70.9 | 74.0 | 95.9 | 24 |
| 68 | $Co_{0.33}$ | 1/3.9/26.1 | 442 | 0.9 | 0.29 | 69.2 | 73.5 | 94.2 | 50 |
| 69 | $Cr_{0.22}$ | 1/4.8/24.7 | 425 | 0.9 | 0.36 | 55.6 | 60.5 | 92.0 | 50 |
| 70 | $Cr_{0.22}$ | 1/4.8/24.7 | 425 | 0.9 | 0.36 | 49.1 | 63.7 | 91.5 | 70 |
| 71 | $Cr_{0.22}$ | 1/4.6/26.1 | 432 | 0.9 | 0.36 | 65.3 | 68.3 | 95.5 | 23 |
| 72 | $Cr_{0.22}$ | 1/5/26.1 | 423 | 0.9 | 0.36 | 67.0 | 67.8 | 98.8 | 36 |
| 73 | $Dy_{0.22}$ | 1/5/25 | 411 | 1.0 | 0.23 | 65.8 | 66.7 | 98.7 | 47 |
| 74 | $Dy_{0.22}$ | 1/5/25 | 417 | 0.9 | 0.23 | 65.6 | 68.5 | 95.7 | 74 |

TABLE V (cont'd.)
$A_aFe_{1.0}P_{1.84}O_x$

| Example No. | Promoter ($A_a$) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 75 | $Eu_{0.22}$ | 1/5/25 | 407 | 0.9 | 0.28 | 63.5 | 65.8 | 96.5 | 24 |
| 76 | $Eu_{0.22}$ | 1/5/25 | 407 | 0.9 | 0.28 | 62.3 | 65.7 | 94.9 | 41 |
| 77 | $Eu_{0.22}$ | 1/4.6/26.1 | 396 | 0.9 | 0.29 | 68.1 | 72.5 | 93.9 | 31 |
| 78 | $Ge_{0.2}$ | 1/4/26 | 412 | 1.7 | 0.22 | 48.5 | 52.6 | 92.3 | |
| 79 | $Ge_{0.2}$ | 1/4/25 | 418 | 1.7 | 0.22 | 54.8 | 57.3 | 95.7 | |
| 80 | $Ho_{0.22}$ | 1/4.8/24.7 | 412 | 0.9 | 0.26 | 58.4 | 61.7 | 94.6 | 40 |
| 81 | $Ho_{0.22}$ | 1/4.8/24.7 | 414 | 0.9 | 0.26 | 54.1 | 63.6 | 85.1 | 57 |
| 82 | $Ho_{0.22}$ | 1/5/26.1 | 434 | 0.9 | 0.27 | 63.4 | 64.2 | 98.7 | 24 |
| 83 | $Ho_{0.22}$ | 1/4.9/26.1 | 424 | 0.9 | 0.27 | 67.3 | 69.0 | 97.6 | 54 |
| 84 | $La_{0.22}$ | 1/5.4/24.7 | 424 | 0.9 | 0.3 | 63.5 | 66.0 | 96.2 | 26 |
| 85 | $La_{0.22}$ | 1/5.4/24.7 | 425 | 0.9 | 0.3 | 64.2 | 66.4 | 96.7 | 30 |
| 86 | $Lu_{0.22}$ | 1/4.8/24.7 | 424 | 0.9 | 0.30 | 59.2 | 62.5 | 94.8 | 27 |
| 87 | $Lu_{0.22}$ | 1/4.8/24.7 | 434 | 0.9 | 0.30 | 53.7 | 63.0 | 85.3 | 50 |

TABLE V (cont'd.)
$A_aFe_{1.0}P_{1.84}O_x$

| Example No. | Promoter ($A_a$) | Feed ratio IBA/Air/$H_2O$ | Temperature °C | Contact Time (sec.) | WWH | Methacrylic acid % Yield | Methacrylic acid % Selectivity | % Conversion | Hours on stream |
|---|---|---|---|---|---|---|---|---|---|
| 88 | $Nd_{0.22}$ | 1/4/23 | 396 | 1.7 | 0.17 | 60.3 | 62.9 | 95.8 | 7 |
| 89 | $Nd_{0.22}$ | 1/4/24 | 402 | 1.7 | 0.17 | 65.4 | 70.3 | 93.1 | 30 |
| 90 | $Ni_{0.33}$ | 1/4.8/24.7 | 432 | 0.9 | 0.33 | 57.2 | 60.7 | 94.2 | 25 |
| 91 | $Ni_{0.33}$ | 1/4.8/26.1 | 428 | 0.9 | 0.32 | 64.7 | 64.8 | 99.8 | 25 |
| 92 | $Ni_{0.33}$ | 1/4.8/26.1 | 418 | 0.9 | 0.32 | 69.1 | 69.7 | 99.2 | 50 |
| 93 | $Ni_{0.33}$ | 1/3.7/26.1 | 413 | 0.9 | 0.32 | 73.2 | 75.1 | 97.5 | 71 |
| 94 | $Sm_{0.22}$ | 1/4.4/25.3 | 425 | 0.9 | 0.27 | 66.7 | 67.6 | 98.7 | 25 |
| 95 | $Sm_{0.22}$ | 1/4.8/25.3 | 432 | 0.9 | 0.27 | 66.2 | 69.0 | 96.0 | 49 |
| 96 | $Ti_{0.2}$ | 1/4/22 | 409 | 1.6 | 0.26 | 63.1 | 66.7 | 94.6 | 6 |
| 97 | $Ti_{0.2}$ | 1/4/28 | 413 | 1.3 | 0.26 | 58.2 | 62.2 | 93.6 | 12 |
| 98[b] | $Th_{0.2}$ | 1/4.1/25.3 | 410 | 1.0 | 0.28 | 75.1 | 75.8 | 99.0 | 23 |
| 99[b] | $Th_{0.2}$ | 1/3.7/25.3 | 410 | 1.0 | 0.28 | 76.3 | 77.4 | 98.6 | 48 |

[b] Molar ratio Fe=1.2

TABLE V (cont'd.)
$A_aFe_{1.0}P_{1.84}O_x$

| Example No. | Promoter ($A_a$) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 100[c] | $Th_{0.2}$ | 1/4.8/25.3 | 417 | 0.9 | 0.34 | 71.6 | 72.6 | 98.6 | 23 |
| 101[c] | $Th_{0.2}$ | 1/3.8/25.3 | 419 | 1.0 | 0.34 | 73.6 | 74.8 | 98.4 | 35 |
| 102[d] | $Th_{0.2}$ | 1/4.8/25.3 | 392 | 1.0 | 0.25 | 68.8 | 68.9 | 99.9 | 24 |
| 103[d] | $Th_{0.2}$ | 1/3.5/25.3 | 382 | 1.0 | 0.24 | 76.2 | 77.2 | 98.6 | 44 |
| 104 | $Tm_{0.22}$ | 1/4.8/24.7 | 412 | 0.9 | 0.27 | 60.1 | 65.3 | 92.0 | 25 |
| 105 | $Tm_{0.22}$ | 1/4.8/24.7 | 423 | 0.9 | 0.27 | 56.4 | 62.7 | 89.9 | 46 |
| 106 | $U_{0.3}$ | 1/3.3/24.7 | 404 | 1.0 | 0.21 | 78.0 | 80.9 | 96.4 | 24 |
| 107 | $U_{0.2}$ | 1/5.4/24.7 | 418 | 0.9 | 0.23 | 71.1 | 71.2 | 99.9 | 25 |
| 108 | $U_{0.2}$ | 1/4.9/24.7 | 394 | 0.9 | 0.23 | 74.2 | 75.0 | 98.9 | 48 |
| 109 | $U_{0.2}$ | 1/5.3/24.7 | 395 | 0.9 | 0.23 | 72.5 | 73.0 | 99.2 | 67 |
| 110 | $U_{0.2}$ | 1/4.7/24.7 | 382 | 1.0 | 0.23 | 75.0 | 76.5 | 98.0 | 73 |
| 111 | $U_{0.2}$ | 1/4.0/24.7 | 388 | 1.0 | 0.23 | 76.1 | 77.7 | 98.0 | 99 |

[c] Molar ratio Fe=0.8
[d] Molar ratio P=1.5

TABLE V (cont'd)

$A_aFe_{1.0}P_{1.84}O_x$

| Example No. | Promoter ($A_a$) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 112 | $V_{0.22}$ | 1/4.8/25 | 391 | 1.0 | 0.41 | 50.1 | 51.1 | 98.1 | 25 |
| 113 | $V_{0.22}$ | 1/4.7/25 | 382 | 1.0 | 0.41 | 49.8 | 52.2 | 95.4 | 45 |
| 114 | $Yb_{0.22}$ | 1/4/23 | 392 | 1.7 | 0.18 | 64.5 | 68.4 | 94.3 | 6 |
| 115 | $Yb_{0.22}$ | 1/4/23 | 396 | 1.7 | 0.18 | 61.1 | 66.3 | 92.2 | 15 |
| 116 | $Zn_{0.33}$ | 1/4/25 | 442 | 1.6 | 0.2 | 58.4 | 60.3 | 96.9 | 10 |
| 117 | $Zr_{0.2}$ | 1/5/23 | 398 | 1.7 | 0.16 | 65.1 | 66.7 | 97.6 | 5 |
| 118 | $Zr_{0.2}$ | 1/4/22 | 398 | 1.7 | 0.16 | 65.6 | 70.0 | 93.7 | 7 |

TABLE VI

Oxydehydrogenation of isobutyric acid to methacrylic acid over $A_aFe_{1.0}P_{1.84}D_dO_x$ catalysts

| Example No. | Promoter & mole ratio ($A_aD_d$) | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 119 | $Ag_{0.4}Mn_{0.4}$ | 1/4.3/26.1 | 400 | 0.9 | 0.29 | 75.1 | 75.4 | 99.6 | 19 |
| 120 | $Ag_{0.4}Mn_{0.4}$ | 1/4.3/26.1 | 385 | 0.9 | 0.29 | 74.7 | 76.4 | 97.8 | 24 |
| 121 | $Ag_{0.4}Mn_{0.4}$ | 1/4.1/36.8 | 385 | 0.7 | 0.29 | 74.1 | 77.8 | 95.3 | 44 |
| 122 | $Ag_{0.5}U_{0.3}$ | 1/4.5/25.3 | 387 | 1.0 | 0.18 | 74.6 | 75.9 | 98.3 | 20 |
| 123 | $Ag_{0.5}U_{0.3}$ | 1/4.5/25.3 | 377 | 1.0 | 0.18 | 73.7 | 76.9 | 95.8 | 23 |
| 124 | $Ag_{0.5}U_{0.3}$ | 1/4.5/25.3 | 382 | 1.0 | 0.18 | 74.2 | 75.8 | 97.9 | 43 |

TABLE VII

Oxydehydrogenation of isobutyric acid to methacrylic acid over $Ag_{0.8}Fe_{1.0}P_{1.84}O_x$ with silica diluents

| Example No. | Weight percent diluent | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| 125 | 20 | 1/4.8/26.1 | 415 | 0.9 | 0.16 | 75.0 | 76.1 | 98.6 | 26 |
| 126 | 20 | 1/4.2/26.1 | 419 | 0.9 | 0.16 | 74.5 | 76.3 | 97.6 | 39 |
| 127 | 20 | 1/4.0/25.3 | 388 | 1.0 | 0.23 | 77.0 | 79.1 | 97.3 | 49 |
| 128 | 30 | 1/4.4/25.3 | 380 | 1.0 | 0.23 | 72.6 | 73.4 | 98.8 | 25 |
| 129 | 30 | 1/3.8/25.3 | 375 | 1.0 | 0.23 | 73.5 | 75.8 | 96.9 | 53 |
| 130 | 40 | 1/4.5/25.3 | 382 | 1.0 | 0.25 | 73.5 | 76.0 | 96.7 | 25 |
| 131 | 40 | 1/3.9/25.3 | 394 | 1.0 | 0.25 | 75.1 | 76.6 | 98.1 | 49 |
| 132 | 50 | 1/4.6/25.3 | 397 | 0.9 | 0.26 | 70.4 | 71.8 | 98.5 | 24 |

TABLE VIII

Oxydehydrogenation of isobutyric acid to methacrylic acid over coated catalysts

| Example No. | Weight % active | Feed ratio IBA/Air/$H_2O$ | Temperature °C | Contact time (sec.) | WWH | Methacrylic acid % Yield | Methacrylic acid % Selectivity | % Conversion | Hours on stream |
|---|---|---|---|---|---|---|---|---|---|
| $Ag_{0.8}Fe_{1.0}P_{1.84}O_x$ on 10—30 mesh (0.60—2.00 mm) alundum | | | | | | | | | |
| 133 | 16.4 | 1/4.1/24.7 | 445 | 0.9 | 0.18 | 76.0 | 77.2 | 98.4 | 23 |
| 134 | 16.4 | 1/3.8/36.8 | 434 | 0.7 | 0.18 | 69.2 | 75.3 | 91.9 | 31 |
| 135 | 30 | 1/3.9/26.1 | 427 | 0.9 | 0.15 | 78.2 | 82.6 | 94.7 | 24 |
| 136 | 30 | 1/4/26.1 | 432 | 0.9 | 0.15 | 79.0 | 81.7 | 96.7 | 36 |
| $Mn_{0.5}Fe_{1.0}P_{1.84}O_x$ on 1/8 inch (0.32 cm) alundum | | | | | | | | | |
| 137 | 35 | 1/5/25.9 | 409 | 0.9 | 0.21 | 69.5 | 72.2 | 96.2 | 23 |
| 138 | 35 | 1/4.7/25.3 | 421 | 0.9 | 0.21 | 66.8 | 67.7 | 98.7 | 48 |
| 139 | 45 | 1/4.8/26.1 | 414 | 0.9 | 0.22 | 64.9 | 66.2 | 98.0 | 26 |
| 140 | 45 | 1/4.2/36 | 430 | 0.7 | 0.22 | 66.6 | 67.4 | 98.9 | 34 |

TABLE IX

Oxydehydrogenation of isobutyric acid to methacrylic acid over 17 weight percent coated $A_aFe_{1.0}P_{1.8}O_x$ on alundum

| Example No. | Promoter and mole ratio | Methacrylic acid % Yield | Methacrylic acid % Selectivity | % Conversion |
|---|---|---|---|---|
| 141 | $Ag_{0.6}$ | 55.1 | 55.1 | 100 |
| 142 | $Cd_{0.3}$ | 50.4 | 50.5 | 99.8 |
| 143 | $Ce_{0.2}$ | 51.3 | 51.3 | 100 |
| 144 | $Dy_{0.2}$ | 54.7 | 55.0 | 99.5 |
| 145 | $La_{0.2}$ | 55.6 | 55.6 | 100 |
| 146 | $Mn_{0.3}$ | 46.1 | 55.5 | 83 |
| 147 | $Ni_{0.3}$ | 44.9 | 44.9 | 100 |
| 148 | $Ti_{0.15}$ | 63.7 | 64.1 | 99.3 |
| 149 | $U_{0.15}$ | 40.6 | 40.8 | 99.6 |
| 150 | $Zr_{0.15}$ | 48.5 | 48.5 | 100 |

**Claims**

1. A catalyst composition represented by the formula

$$A_aFe_bP_cD_dO_x$$

wherein A is selected from Cd, Cr, Ge, Te, Th, Ti, U, V, Zr, rare earths and mixtures thereof;

wherein D is selected from Ag, Cu, Mn and mixtures thereof;

and wherein

a=0—1.0;

b=0.75—1.5;

c=1.0—2.0; and

d=0—2.0; provided that a+d is greater than zero and when D is Ag or a mixture of Ag and Cu a is greater than zero;

and

x is the number of oxygens needed to satisfy the valence requirements of the remaining elements.

2. A catalyst as claimed in Claim 1 characterised in that the rare earths are selected from La, Ce, Nd, Sm, Eu, Dy, Ho, Tm, Yb, and Lu.

3. A catalyst as claimed in Claim 1 or Claim 2 characterised in that a is 0.15 to 0.5.

4. A catalyst as claimed in any of Claims 1 to 3 characterised in that d is 0.2 to 1.5.

5. A catalyst as claimed in any of Claims 1 to 4 in which D represents at least silver.

6. A catalyst as claimed in any of Claims 1 to 4 characterised in that D represents at least manganese.

7. A catalyst as claimed in any of Claims 1 to 4 characterised in that D represents at least copper.

8. A catalyst claimed in Claim 1 characterised in that A represents at least thorium.

9. A catalyst claimed in Claim 1 characterised in that A represents at least uranium.

10. A catalyst as claimed in Claim 1 characterised in that A represents at least one rare earth element.

11. A catalyst as claimed in any of Claims 1 to 10 characterised in that it additionally contains inert diluents.

12. A catalyst as claimed in any of Claims 1 to 11 characterised in that it is coated upon a support.

13. A process for the oxydehydrogenation of saturated carboxylic acids to form alpha, beta unsaturated carboxylic acids comprising contacting said saturated acids with molecular oxygen or an oxygen-containing gas in the vapour phase, at a reaction temperature of 250°C to 600°C in the presence of a catalyst having the empirical formula

$$A_aFe_bP_cD_dO_x$$

wherein A is selected from Al, B, Be, Cd, Co, Cr, Ga, Ge, In, Ni, Te, Th, Ti, Tl, U, V, Zn, Zr, rare earths and mixtures thereof, wherein D is selected from Ag, Cu, Mn and mixtures thereof,

and wherein

a=0—1.0
b=0.75—1.5
c=1.0—2.0
d=0—2.0
a+d is greater than zero

and

x is the number of oxygens needed to satisfy the valence requirements of the remaining elements.

14. A process for the oxydehydrogenation of saturated carboxylic acids to form alpha, beta unsaturated carboxylic acids comprising contacting said saturated acids with molecular oxygen or an oxygen containing gas in the vapour phase at a reaction temperature of from 250°C to 600°C in the presence of a catalyst as claimed in any of Claims 1 to 12.

15. A process as claimed in claim 13 or claim 14 characterised in that the saturated acid is represented by the formula

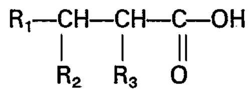

wherein $R_1$, $R_2$, and $R_3$ are each independently selected from hydrogen and alkyl groups containing from 1 to 4 carbon atoms.

16. A process as claimed in Claim 15 characterised in that the acid comprises isobutyric acid.

17. A process as claimed in any of Claims 13 to 16 characterised in that the saturated acid is additionally contacted with an inert diluent gas.

18. A process as claimed in any of Claims 13 to 16 characterised in that said saturated acid is additionally contacted with steam.

19. A process as claimed in any of Claims 13 to 18 characterised in that the reaction temperature is within the range of 325°C to 450°C.

20. A process as claimed in any of Claims 13 to 19 characterised in that the reaction is conducted at a pressure greater than atmospheric pressure.

21. A process for the preparation of catalysts as claimed in Claim 1 wherein the constituents of the catalyst are prepared by introducing a compound of iron and a compound containing the promoter element into a liquid medium, such as water and contacting with the phosphorus compound, or introducing them into a solution of phosphoric acid in a liquid medium, such as water and recovering the catalyst by drying and calcining.

**Patentansprüche**

1. Ein Katalysatorprodukt entsprechend der Formel

$$A_aFe_bP_cD_dO_x$$

worin A ausgewählt ist aus der Gruppe Cd, Cr, Ge, Te, Th, Ti, U, V, Zr, die Seltenen Erden und Gemischen hiervon; D ausgewählt ist aus der Gruppe Ag, Cu, Mn und Gemische hiervon;

und worin

a=0 bis 1,0,
b=0,75 bis 1,5,
c=1,0 bis 2,0 und
d=0 bis 2 mit der Maßgabe, daß a+d größer als 0 und, wenn D Ag ist oder ein Gemisch von Ag und Cu ist, a größer als 0 ist,

und

x die Zahl der Sauerstoffatome, die notwendig ist, um die Wertigkeitserfordernisse der übrigen Elemente zu erfüllen.

2. Katalysator gemäß Anspruch 1, dadurch gekennzeichnet, daß die Seltenen Erden ausgewählt sind aus der Gruppe La, Ce, Nd, Sm, Eu, Dy, Ho, Tm, Yb und Lu.

3. Katalysator gemäß Anspruch 1 oder 2, dadurch gekennzeichnet, daß a 0,15 bis 0,5 ist.

4. Katalysator gemäß einem der Ansprüche 1 bis 3, dadurch gekennzeichnet, daß d 0,2 bis 1,5 ist.

5. Katalysator gemäß einem der Ansprüche 1 bis 4, dadurch gekennzeichnet, daß D mindestens Silber darstellt.

6. Katalysator gemäß einem der Ansprüche 1 bis 4, dadurch gekennzeichnet, daß D mindestens Mangan darstellt.

7. Katalysator gemäß einem der Ansprüche 1 bis 4, dadurch gekennzeichnet, daß D mindestens Kupfer darstellt.

8. Katalysator gemäß Anspruch 1, dadurch gekennzeichnet, daß A mindestens Thorium ist.

9. Katalysator gemäß Anspruch 1, dadurch gekennzeichnet, daß A mindestens Uran ist.

10. Katalysator gemäß Anspruch 1, dadurch gekennzeichnet, daß A mindestens eine der Seltenen Erden darstellt.

11. Katalysator gemäß einem der Ansprüche 1 bis 10, dadurch gekennzeichnet, daß er zusätzlich inerte Verdünnungsstoffe enthält.

12. Katalysator gemäß einem der Ansprüche 1 bis 11, dadurch gekennzeichnet, daß er auf einem Trägermaterial aufgeschichtet ist.

13. Verfahren zur Oxydehydrogenierung gesättigter Carbonsäuren zur Bildung von α,ß-ungesättigten Carbonsäuren, dadurch gekennzeichnet, daß die gesättigten Säuren mit molekularem Sauerstoff oder einem sauerstoffhaltigen Gas in der Dampfphase bei einer Reaktionstemperatur von 250 bis 600°C in Anwesenheit eines Katalysators in Berührung gebracht wird, der die empirische Formel

$$A_aFe_bP_cD_dO_x$$

hat,

worin A ausgewählt ist aus der Gruppe Al, B, Be, Cd, Co, Cr, Ga, Ge, In, Ni, Te, Th, Ti, Tl, U, V, Zn, Zr, die Seltenen Erden und Gemische hiervon und D ausgewählt ist aus der Gruppe Ag, Cu, Mn und Gemischen hiervon und worin

a=0 bis 1,0,
b=0,75 bis 1,5,
c=1,0 bis 2,0,
d=0 bis 2,0
a+d größer als 0, und
x die Zahl der Sauerstoffatome ist, die zur Absättigung der Wertigkeitsanforderungen der übrigen Elemente notwendig ist.

14. Verfahren zur Oxydehydrogenierung gesättigter Carbonsäuren unter Bildung von α,ß-ungesättigten Carbonsäuren, dadurch gekennzeichnet, daß die gesättigte Säure mit molekularem Sauerstoff oder einem sauerstoffhaltigen Gas in der Dampfphase bei einer Temperatur von 250 bis 600°C in Anwesenheit eines Katalysators gemäß einem der Ansprüche 1 bis 12 in Berührung gebracht wird.

15. Verfahren gemäß Anspruch 13 oder 14, dadurch gekennzeichnet, daß die gesättigte Säure der Formel

$$R_1\text{—}\underset{\displaystyle R_2}{\underset{|}{CH}}\text{—}\underset{\displaystyle R_3}{\underset{|}{CH}}\text{—}\underset{\displaystyle O}{\underset{\|}{C}}\text{—OH}$$

entspricht, worin $R_1$, $R_2$ und $R_3$ jeder und unabhängig voneinander aus der Gruppe Wasserstoff und den Alkylgruppen mit 1 bis 4 Kohlenstoffatomen ausgewählt ist.

16. Verfahren gemäß Anspruch 15, dadurch gekennzeichnet, daß die Säure Isobuttersäure umfaßt.

17. Verfahren gemäß einem der Ansprüche 13 bis 16, dadurch gekennzeichnet, daß die gesättigte Säure zusätzlich mit einem inerten Verdünnungsgas in Berührung gebracht wird.

18. Verfahren gemäß einem der Ansprüche 13 bis 16, dadurch gekennzeichnet, daß die Säure zusätzlich mit Dampf in Berührung gebracht wird.

19. Verfahren gemäß einem der Ansprüche 13 bis 18, dadurch gekennzeichnet, daß die Reaktionstemperatur im Bereich von 325 bis 450°C liegt.

20. Verfahren gemäß einem der Ansprüche 13 bis 19, dadurch gekennzeichnet, daß die Reaktion bei einem Druck größer als atmosphärischer Druck durchgeführt wird.

21. Verfahren zur Herstellung von Katalysatoren gemäß Anspruch 1, dadurch gekennzeichnet, daß die Bestandteile des Katalysators hergestellt werden, in dem man eine Eisenverbindung und eine Verbindung der Promotorelemente in eine Flüssigkeit wie Wasser einführt, sie mit einer Phosphorverbindung in Berührung gebracht werden, sie zum Beispiel in eine Lösung von Phosphorsäure in einem flüssigen Medium wie Wasser eingeführt werden und der Katalysator durch Trocknen und Glühen gewonnen wird.

**Revendications**

1. Composition de catalyseur représentée par la formule:

$$A_aFe_bP_cD_dO_x$$

dans laquelle A est choisi parmi Cd, Cr, Ge, Te, Th, Ti, U, V, Zr, les terres rares et leurs mélanges; dans laquelle D est choisi parmi Ag, Cu, Mn et leurs mélanges; et dans laquelle

a=0—1,0;
b=0,75—1,5;
c=1,0—2,0; et
d=0—2,0; à condition que a+b soit plus grand que zéro et que, lorsque D est Ag ou un mélange de Ag et Cu, a soit plus grand que zéro;
et
x est le nombre d'atomes d'oxygène nécessaires pour satisfaire les exigences de valence des éléments restants.

2. Catalyseur selon la revendication 1, caractérisé par le fait que les terres, rares sont choisies parmi La, Ce, Nd, Sm, Eu, Dy, Ho, Tm, Yb, et Lu.

3. Catalyseur selon la revendication 1 ou la revendication 2, caractérisé par le fait que a va de 0,15 à 0,5.

4. Catalyseur selon l'une des revendications 1 à 3, caractérisé par le fait que d va de 0,2 à 1,5.

5. Catalyseur selon l'une des revendications 1 à 4, dans lequel D représente au moins l'argent.

6. Catalyseur selon l'une des revendications 1 à 4, caractérisé par le fait que D représente au moins le manganèse.

7. Catalyseur selon l'une des revendications 1 à 4, caractérisé par le fait que D représente au moins le cuivre.

8. Catalyseur selon la revendication 1, caractérisé par le fait que A représente au moins le thorium.

9. Catalyseur selon la revendication 1, caractérisé par le fait que A représente au moins l'uranium.

10. Catalyseur selon la revendication 1, caractérisé par le fait que A représente au moins un élément des terres rares.

11. Catalyseur selon l'une des revendications 1 à 10, caractérisé par le fait qu'il contient en outre des diluants inertes.

12. Catalyseur selon l'une des revendications 1 à 11, caractérisé par le fait qu'il est appliqué sur un support.

13. Procédé d'oxydéshydrogénation des acides carboxyliques saturés pour former des acides carboxyliques alpha, bêta-insaturés, consistant à mettre lesdits acides saturés en contact avec de l'oxygène moléculaire ou avec un gaz contenant de l'oxygène, en phase vapeur, à une température de réaction de 250°C à 600°C, en présence d'un catalyseur ayant pour formule empirique:

$$A_aFe_bP_cD_dO_x$$

dans laquelle A est choisi parmi Al, B, Be, Cd, Co, Cr, Ga, Ge, In, Ni, Te, Th, Ti, Tl, U, V, Zn, Zr, les terres rares et leurs mélanges, dans laquelle D est choisi parmi Ag, Cu, Mn et leurs mélanges, et dans laquelle:

a=0—1,0
b=0,75—1,5
c=1,0—2,0
d=0—2,0
a+d est plus grand que zéro et
x est le nombre d'atomes d'oxygène nécessaires pour satisfaire les exigences de valence des éléments restants.

14. Procédé d'oxydéshydrogénation d'acides carboxyliques saturés pour former des acides carboxyliques alpha, bêta-insaturés, consistant à mettre lesdits acides saturés en contact avec le l'oxygène moléculaire ou avec un gaz contenant de l'oxygène, en phase vapeur, à une température de réaction de 250°C à 600°C, en présence d'un catalyseur tel que revendiqué à l'une des revendications 1 à 12.

15. Procédé selon la revendication 13 ou la revendication 14, caractérisé par le fait que l'acide saturé est représenté par la formule

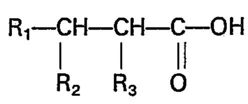

dans laquelle $R_1$, $R_2$ et $R_3$ sont choisis chacun, indépendamment, parmi l'hydrogène et les radicaux alkyle contenant de 1 à 4 atomes de carbone.

16. Procédé selon la revendication 15, caractérisé par le fait que l'acide comprend l'acide isobutyrique.

17. Procédé selon l'une des revendications 13 à 16, caractérisé par le fait qu'on met en outre l'acide saturé en contact avec un gaz diluant inerte.

18. Procédé selon l'une des revendications 13 à 16, caractérisé par le fait qu'on met en outre l'acide saturé en contact avec de la vapeur d'eau.

19. Procédé selon l'une des revendications 13 à 18, caractérisé par le fait que la température de réaction se situe dans l'intervalle de 325°C à 450°C.

20. Procédé selon l'une des revendications 13 à 19 caractérisé par le fait qu'on mène la réaction à une pression supérieure à la pression atmosphérique.

21. Procédé de préparation de catalyseurs tels que revendiques à la revendication 1, dans lequel on prépare les constituants du catalyseur en introduisant un composé du fer et un composé contenant l'élément promoteur dans un milieu liquide, tel que l'eau, et en les mettant en contact avec le composé du phosphore, ou bien en les introduisant dans une solution d'acide phosphorique dans un milieu liquide, tel que l'eau, et en récupérant le catalyseur par séchage et calcination.